# EUROPÄISCHE PATENTSCHRIFT

(11) **EP 1 357 190 B1**
(45) Veröffentlichungstag und Bekanntmachung des Hinweises auf die Patenterteilung: **29.06.2011**
(21) Anmeldenummer: 03007098.1
(22) Anmeldetag: 28.03.2003
(51) Int. Cl.: C12N 15/79, C12N 1/11, C12N 15/82, C12N 1/00

(54) **Verfahren und Marker zur einfachen Transformation und Selektion von rekombinanten Ciliaten**
Methods and markers for simple transformation and selection of recombinant ciliates
Procédés et marqueurs pour la transformation et la sélection simple des ciliates recombinant

(30) Priorität: 30.03.2002 DE 10214406
(43) Veröffentlichungstag der Anmeldung: 29.10.2003
(73) Patentinhaber: Lonza Ltd., 4002 Basel (CH)
(72) Erfinder: Rüsing, Matthias, Dr., 50935 Köln (DE)
(74) Vertreter: Michalski Hüttermann & Partner Patentanwälte

(56) Entgegenhaltungen:
- EP-A- 1 130 103
- WO-A-00/46381
- WO-A-00/52176
- WO-A-01/20000
- NAKASHIMA S ET AL: "MOLECULAR CLONIG OF DELTA 9 FATTY ACID DESATURASE FROM THE PROTOZOAN TETRAHYMENA THERMOPHILA AND ITS MRNA EXPRESSION DURING THERMAL MEMBRANE ADAPTATION" BIOCHEMICAL JOURNAL, THE BIOCHEMICAL SOCIETY, LONDON, GB, Bd. 317, Nr. 1, 1996, Seiten 29-34, XP001022408 ISSN: 0264-6021
- TURKEWITZ A P ET AL: "Functional genomics: the coming of age for Tetrahymena thermophila" TRENDS IN GENETICS, ELSEVIER, AMSTERDAM, NL, Bd. 18, Nr. 1, 1. Januar 2002 (2002-01-01), Seiten 35-40, XP004326534 ISSN: 0168-9525
- GAERTIG ET AL: "High frequency vector-mediated transformation and gene replacement in Tetrahymena" NUCLEIC ACIDS RESEARCH, OXFORD UNIVERSITY PRESS, SURREY, GB, Bd. 22, Nr. 24, 1994, Seiten 5391-5398, XP002128557 ISSN: 0305-1048
- SHANG YUHUA ET AL: "A robust inducible-repressible promoter greatly facilitates gene knockouts, conditional expression, and overexpression of homologous and heterologous genes in Tetrahymena thermophila" PROCEEDINGS OF THE NATIONAL ACADEMY OF SCIENCES OF USA, NATIONAL ACADEMY OF SCIENCE. WASHINGTON, US, Bd. 99, Nr. 6, 19. März 2002 (2002-03-19), Seiten 3734-3739, XP002208981 ISSN: 0027-8424
- ADL S.M. ET AL: 'The New Higher Level Classification of Eukaryotes with Emphasis on the Taxonomy of Protists' JOURNAL OF EUKARYOTIC MICROBIOLOGY, LAWRENCE, KS, US LNKD- DOI:10.1111/J.1550-7408.2005.00053.X Bd. 52, Nr. 5, 01 Januar 2005, Seiten 399 - 451, XP003023667 ISSN: 1066-5234
- "Phylum Ciliophora - Conjugating Ciliated Protists with Nuclear Dualism"; "Chapter 4" In: Denis H. Lynn (Ed.): "The Ciliated Protozoa", 2008, Springer ISBN: 978-1-4020-8238-2 page p. 89,
- THEODOR DINGERMANN (ED.): 'Gentechnik Biotechnik', 1999, WISSENSCHAFLICHE VERLAGSGESELLSCHAFT Seiten 131-133 - 163
- HILL ET AL: "Modulation of membrane fluidity in a fatty acid auxotrophe of Tetrahymena thermophila", BIOCHIMICA ET BIOPHYSICA ACTA. BIOMEMBRANES, AMSTERDAM, NL LNKD- DOI:10.1016/0005-2736(80)90256-4, vol. 595, no. 1, 12 January 1980 (1980-01-12), pages 140-145, XP023361473, ISSN: 0005-2736 [retrieved on 1980-01-12]
- SANFORD Y.M. ET AL: 'PHENYLKETONURIC TETRAHYMENA: PHENYLALANINE HYDROXYLASE MUTANTS AND OTHER TYROSINE AUXOTROPHS' PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.78.12.7614 Bd. 78, Nr. 12, 01 Dezember 1981, Seiten 7614 - 7618, XP009014822 ISSN: 0027-8424
- HEPFER C.E. ET AL: 'Enrichment for Auxotrophic mutants of Tetrahymena using density gradients centrifugation' JOURNAL OF PROTOZOOLOGY, LAWRENCE, KANSAS, US Bd. 27, Nr. 4, 01 Januar 1980, Seiten 431 - 434, XP003023670 ISSN: 0022-3921
- MITTERBAUER R. ET AL: 'Saccharomyces cerevisiae URH1 (encoding Uridine-cytidine N-Ribohydrolase): Functional complementation by a nucleoside hydrolase from a protozoan parasite and by a mammalian uridine phosphorylase' APPLIED AND ENVIRONMENTAL MICROBIOLOGY, AMERICAN SOCIETY FOR MICROBIOLOGY, US LNKD- DOI:10.1128/AEM.68.3.1336-1343.2002 Bd. 68, Nr. 3, 01 März 2002, Seiten 1336 - 1343, XP003023671 ISSN: 0099-2240
- Anonymous: "Phylogenetic Tree of Life", , 12 May 2009 (2009-05-12), Retrieved from the Internet: URL:http://en.wikipedia.org/wiki/Phylogene tic_tree [retrieved on 2009-05-12]

## Beschreibung

Die vorliegende Erfindung betrifft ein Verfahren zur Herstellung von genetisch modifizierten (rekombinanten) Ciliaten ohne den Einsatz von Negativselektionsmarkern und ein effizientes Verfahren zur Herstellung von Proteinen mittels dergestalt modifizierter Protisten.

Die Produktion rekombinanter Proteine durch heterologe Proteinexpression stellt eine Alternative zur Gewinnung von Proteinen aus natürlichen Quellen dar. Natürliche Quellen für Proteine, die beispielsweise als Pharmazeutika dienen, sind oft limitiert, sehr teuer in der Reinigung oder stehen einfach nicht zur Verfügung. Außerdem können sie durch die Gefahr von toxischen oder insbesondere infektiösen Verunreinigungen sehr problematisch sein. Die Biotechnologie ermöglicht dagegen heute eine wirtschaftliche und sichere Produktion von einer ganzen Reihe von Proteinen in ausreichenden Mengen mittels heterologer Expression für verschiedenste Einsatzgebiete: z.B.: Antikörper (für die Diagnose, passive Immunisierung und Forschung), Hormone (wie Insulin, Erythropoietin (EPO), Interleukine usw. für den therapeutischen Einsatz), Enzyme (z.B. für den Einsatz in der Lebensmitteltechnologie, Diagnose, Forschung), Blutfaktoren (zur Behandlung von Hämophilie), Impfstoffe usw. (Glick & Pasternak 1998, Molecular Biotechnology, ASM Press, Washington DC, Chapter 10: 227-252).

Proteine für einen medizinischen Einsatz, besonders humane Proteine, müssen in ihren biochemischen, biophysikalischen und funktionalen Eigenschaften identisch mit dem natürlichen Protein sein. Bei einer rekombinanten Herstellung solcher Proteine durch heterologe Genexpression ist dabei zu beachten, dass es in eukaryotischen Zellen im Gegensatz zu Bakterien eine Reihe von posttranslationalen Proteinmodifizierungen gibt: Ausbildung von Disulfidbrücken, proteolytische Spaltung von Vorläuferproteinen, Modifizierungen von Aminosäureresten (Phosphorylierung, Acetylierung, Acylierung, Sulfatisierung, Carboxylierung, Myristylierung, Palmitylierung und insbesondere Glycosylierungen). Darüber hinaus werden Proteine in eukaryotischen Zellen oft erst durch einen komplexen Mechanismus unter Beteiligung von Chaperonen in die richtige dreidimensionale Struktur gebracht.

Diese Modifizierungen spielen eine sehr wichtige Rolle hinsichtlich der spezifischen strukturellen und funktionalen Eigenschaften der Proteine, wie etwa der Aktivität von Enzymen, der Spezifität (Rezeptorbindung, Zellerkennung), Faltung, Löslichkeit etc. des Proteins (Ashford & Platt 1998, in: Post-Translational Processing - A Practical Approach, Ed. Higgins & Hames, Oxford University Press, Chapter 4: 135-174; Glick & Pasternak 1998, Molecular Biotechnology, ASM Press, Washington DC, Chapter 7: 145-169).

Von der natürlichen Struktur abweichende Modifizierungen können beispielsweise zu Inaktivierung der Proteine führen oder ein hohes allergenes Potential besitzen.

Obwohl eine ganze Reihe von bakteriellen und eukaryotischen Expressionssystemen für die Produktion von rekombinanten Proteinen etabliert sind, gibt es kein universelles System, welches dass ganze Spektrum der - insbesondere bei eukaryotischen Proteinen - möglichen Proteinmodifikationen abdeckt und damit universell einsetzbar wäre (Castillo 1995, Bioprocess Technology 21: 13-45; Geise et al. 1996, Prot. Expr. Purif. 8: 271-282; Verma et al. 1998 J. Immunological Methods 216: 165-181; Glick & Pasternak 1998, Molecular Biotechnology, ASM Press, Washington DC, Chapter 7: 145-169). Eine weiteres Problem besteht darin, dass einige der vielfach genutzen Systeme ungewöhnliche und teilweise unerwünschte posttranslationale Proteinmodifikationen einführen. So sind rekombinant exprimierte Proteine aus Hefen mitunter extrem stark mit Mannoseresten modifiziert. Diese sogenannten "high Mannose" Strukturen aus Hefen bestehen dabei aus ca. 8-50 Mannoseresten und unterscheiden sich damit erheblich von den mannosereichen Glycoproteinstrukturen aus Säugetierzellen, die maximal 5-9 Mannosereste aufweisen (Moremen et al. 1994, Glycobiology 4(2): 113-125). Diese hefetypischen Mannosestrukturen sind starke Allergene und somit bei der Produktion von rekombinanten Glycoproteinen für einen therapeutischen Einsatz sehr problematisch (Tuite et al. 1999, in Protein Expression - A Practical Approach, Ed. Higgins & Hames, Oxford University Press, Chapter 3: insbesondere Seite 76). Außerdem können in Hefen keine hybriden oder komplexen Glycoproteinstrukturen gebildet werden, was deren Einsatz als Expressionssystem weiter einschränkt.

Pflanzen, die in letzter Zeit immer öfter als Produktionssysteme für rekombinante Proteine diskutiert und genutzt werden, weisen dagegen statt der für Säuger typischen Sialinsäure auf den Glykoproteinstrukturen Xylose auf (Ashford & Platt, s.o.). Xylose und die ebenfalls bei Pflanzen nachgewiesene α-1,3 verknüpfte Fucose können ein allergisches Risiko darstellen und sind somit problematisch (Jenkins et al. 1996, Nature Biotech. 14: 975-981).

Deshalb besteht gerade für neue, alternative eukaryotische Expressionssysteme ein großer Bedarf, vor allem als Alternative zu der sehr kostenintensiven und aufwendigen Herstellung von rekombinanten Proteinen mittels Säugerkulturzellen.

Ein solches System sollte idealerweise folgende Anforderungen erfüllen: Es müssen 1) Selektionsmarker und regulative DNA-Elemente (wie Transkriptions- und Translationssignale etc.) zur Verfügung stehen. 2) Das Expressionssystem sollte wichtige eukaryotische posttranslationale Proteinmodifizierungen ermöglichen und 3) die Herstellung der rekombinanten Proteine sollte möglichst einfach und wirtschaftlich sein, z.B. durch die Möglichkeit der Fermentation der Zellen oder Organismen im Produktionsmaßstab (beispielsweise mehrere 1000 L) auf einfachen Medien und einfache Aufarbeitung der Produkte.

Zu den bereits etablierten eukaryotischen Expressionssystemen wie z.B. Hefe-Säuger- oder Insektenkulturzellen könnten Protozoen oder Protisten (als Definition siehe z.B. Hendersons's dictionary of biological terms, 10th ed. 1989, Eleanor Lawrence, Longman Scientific & Technical, England, oder Margulis et al. (Eds) 1990. Handbook of Protoctista, Jones & Bartlett, Boston; van den Hoek et al. 1995, Algae - An introduction to phycology, Cambridge University Press) eine interessante Alternative darstellen. Bei diesen Organismen handelt es sich um eine sehr heterogene Gruppe von eukaryotischen, in der Regel einzelligen Mikroorganismen. Sie besitzen die für eukaryotische Zellen typische Kompartimentierung und Differenzierung. Einige sind relativ nah verwandt mit höheren Eukaryoten, ähneln andererseits aber betreffend Kultivierung und Wachstum eher Hefen oder sogar Bakterien und lassen sich relativ leicht bei hoher Zelldichte auf einfachen Medien im großen Maßstab fermentieren.

Ein für die Expression heterologer Proteine interessanter Protist ist beispielsweise der Ciliat *Tetrahymena*, insbesondere *Tetrahymena thermophila*. Es handelt sich dabei um einen nicht pathogenen, einzelligen, eukaryotischen Mikroorganismus, der relativ nah verwandt zu höheren Eukaryoten ist und die dafür typischen Zelldifferenzierungen aufweist. Die posttranslationalen Protein-Modifizierungen in *Tetrahymena* ähneln denen in Säugerzellen stärker, als z.B. die in Hefe oder anderen eukaryotischen Expressionssystemen nachgewiesenen. Beispielsweise findet man bei *Tetrahymena* keine stark antigenen Zuckerketten auf Glykoproteinen, wie in Hefe ("high-Mannose"-Strukturen), und pflanzlichen oder auf niederen Tierzellkulturen basierenden Expressionssystemen (Xylosereste) (siehe oben). Obwohl *Tetrahymena* ein echter, komplex differenzierter Eukaryot ist, ähnelt er in seinen Kultivierungs- und Wachstumseigenschaften eher den einfachen Hefen oder Bakterien und lässt sich gut auf relativ billigen Magermilchmedien im großen Maßstab fermentieren. Unter optimalen Bedingungen beträgt die Generationszeit etwa 1,5 - 3 h und es können sehr hohe Zelldichten (2,2 x 10⁷ Zellen/ml, entsprechend 48 g/l Biotrockenmasse) erreicht werden (Kiy und Tiedke 1992, Appl. Microbiol. Biotechnol. 37: 576-579; Kiy und Tiedke 1992, Appl. Microbiol. Biotechnol. 38: 141-146). Dadurch ist *Tetrahymena* für eine fermentative Produktion von rekombinanten Proteinen im großen Maßstab sehr interessant.

Ein weiterer vorteilhafter Aspekt von *Tetrahymena* als Expressionssystem ist die Tatsache, dass in *Tetrahymena* eine Integration des heterologen Gens durch homologe DNA-Rekombination möglich ist. Dadurch können mitotisch stabile Transformanten erzeugt werden. Durch die homologe DNA-Rekombination sind darüber hinaus auch gezielte Gen-Ausschaltungen ("Knock-outs") möglich (Bruns & Cassidy-Hanley in: Methods in Cell Biology, Volume 62, Ed. Asai & Forney, Academic Press (1999) 501-512; Hai et al. in: Methods in Cell Biology, Volume 62, Ed. Asai & Forney, Academic Press (1999) 514-531; Gaertig et al. (1999) Nature Biotech. 17: 462-465 oder Cassidy-Hanley et al. 1997 Genetics 146:135-147). Außerdem kann wahlweise der somatische Makronucleus oder der generative Mikronucleus transformiert werden. Bei der Makronucleustransformation erhält man sterile Transformanten, was aus Sicherheits- oder Akzeptanzfragen vorteilhaft sein kann.

Die Transformation von *Tetrahymena* kann durch Mikroinjektion, Elektroporation oder Mikropartikelbeschuss erreicht werden. Dafür stehen eine Reihe von Vektoren, Promotoren etc. zur Verfügung. Die Selektion der Transformanten erfolgt üblicherweise mittels eines Resistenzmarkers. So wurde *Tetrahymena* z. B. erfolgreich mit einem rDNA-Vektor transformiert. Selektion erfolgte in diesem Fall durch eine Paromomycin-Resistenz-Mutation der rRNA (Tondravi et al. 1986, PNAS 83:4396; Yu et al. 1989, PNAS 86: 8487-8491). In weiteren Transformationsexperimenten wurden erfolgreich Cycloheximid- oder Neomycin-Resistenzen in *Tetrahymena* exprimiert (Yao et al. 1991, PNAS 88:9493-9497; Kahn et al. 1993, PNAS 90: 9295-9299). Neben diesen Markergenen haben Gaertig et al. (1999, Nature Biotech. 17: 462-465) erfolgreich zwei rekombinante Proteine in *Tetrahymena* exprimiert (ein Fischparasiten-Antigen und ein partielles Ovalbumin aus dem Huhn). Die Selektion erfolgte mit Hilfe von Paclitaxel (Taxol). Dieses von Gaertig et al. entwickelte System ist zum Patent angemeldet (WO 00/46381).

Nur für einige wenige weitere Protisten oder Protozoen wurden Verfahren zur Transformation und heterologen Proteinexpression beschrieben. Als weiterer Ciliat sei hier *Paramecium* (Boileau et al. 1999, J. Eukaryot. Microbiol. 46: 56-65) erwähnt. Verschiedene Versuche zur Transformation und Expression rekombinanter Proteine wurden aber auch in parasitischen Protozoen, wie *Trypanosoma, Leishmania, Plasmodium* u. a. unternommen (Beverly 2000, WO 00/58483). Eine Übersicht darüber gibt Kelly (1997, Advances in Parasitology, Vol 39, 227-270). Eine Möglichkeit zur heterologen Proteinexpression wurde auch im Schleimpilz *Dictyostelium discoideum* gezeigt (Manstein et al. 1995, Gene 162: 129-134, Jung und Williams 1997, Biotechnol. Appl. Biochem. 25: 3-8) aber auch in photoautotrophen Protisten (Mikroalgen) wie *Chlamydomonas* (Hall et al. 1993, Gene 124: 75-81), *Volvox* (Schiedlmeier et al. 1994, PNAS 91: 5080-5084), bestimmten Dinoflagellaten (ten Lohuis & Miller 1998, Plant Journal 13: 427-435) und Diatomeen (Dunahay et al. 1995, J. Phycol. 31: 1004-1012). In den meisten Fällen wurden dabei jedoch lediglich einfache Resistenzmarker oder nicht-humane Selektionsmarker exprimiert.

Keiner dieser Organismen wurde bisher in größerem Maßstab für die Produktion von rekombinanten Proteinen eingesetzt. Ein großes Problem für viele dieser und weiterer, möglicherweise interessanter Systeme ist das Fehlen gut etablierter gentechnologischer Methoden und insbesondere von molekularbiologischen "Werkzeugen", wie beispielsweise Vektoren, Markern etc.

Dabei ist das Vorhandensein eines selektiven Markers eine notwendige Voraussetzung, um Zellen gezielt genetisch modifizieren zu können. In der Regel erfolgt die Selektion transformierter Zellen oder Organismen durch Negativselektionsmarker, meist die Resistenz gegen ein Antibiotikum (z.B. Ampicillin, Kanamycin, Tetracyclin, Neomycin usw.). Selten kommt die Selektion durch Einbringen eines essentiellen Gens in einen - dieses Genprodukt betreffenden- defekten Zelltyp zum Einsatz (positive Selektion oder Selektion durch Komplementierung). Dazu gehören z.B. LEU- oder URA3- basierte Selektionssysteme bei Hefen (siehe Glick & Pasternak, Molecular Biotechnology, Principles and Applications of Recombinant DNA, 1998, 2nd ed., ASM Press, Washington, D.C. Seiten 109-169). Für die noch wenig untersuchten "neuen Organismen" wie die bereits genannten Protisten, steht dieser zweite Ansatz jedoch nicht zur Verfügung. Dadurch ist die Nutzbarkeit dieser Systeme, vor allem für die Herstellung rekombinanter Proteine im Produktionsmaßstab, stark eingeschränkt.

Die negative Selektion birgt generell gravierende Nachteile. Zum einen muss für das gewünschte Produkt unnötige DNA in den Produktionsorganismus eingebracht werden, was Bedenken hinsichtlich der biologischen Sicherheit, aber vor allem der öffentlichen Akzeptanz hervorrufen kann. Zum anderen müssen die Organismen zur Aufrechterhaltung des Selektionsdrucks während der gesamten Produktionszeit in Gegenwart der entsprechenden Antibiotika kultiviert werden, was die Kosten ev. enorm in die Höhe treibt. Es sind nicht nur die Kosten für das Antibiotikum selbst, bzw. die Entsorgung der Produktionsabfälle (Medien, ...) zu berücksichtigen, sondern auch die Aufarbeitung der Proteine kann sich ungleich schwieriger gestalten. Aber abgesehen von Wirtschaftlichkeit sind Umweltverträglichkeit, biologische, bzw. gentechnologische Sicherheit und wiederum vor allem die öffentliche Akzeptanz als sehr problematisch einzustufen. Zudem stellen sich Probleme rein technischer Natur, wenn der Organismus mehrfach transformiert werden muss. Die gleichzeitige Gegenwart mehrer Antibiotika bzw. die gleichzeitige Expression meherer Antibiotikumresistenzgene kann sich, sofern überhaupt möglich, äußerst nachteilig auf die Organsimen auswirken, bzw. zu ungeahnten Nebeneffekten führen.

In Anbetracht des Standes der Technik war es nun Aufgabe der vorliegenden Erfindung, ein Verfahren zur positiven Selektion genetisch modifizierter Protisten zur Verfügung zu stellen, das unter anderem eine effiziente Produktion von rekombinanten Proteinen ermöglicht.

Gelöst wird diese, sowie weitere nicht explizit genannte Aufgaben, die jedoch aus den hierin einleitend diskutierten Zusammenhängen ohne weiteres ableitbar oder erschließbar sind, durch die in den Patentansprüchen definierten Ausführungformen der vorliegenden Erfindung.

Ein Verfahren zur Herstellung rekombinanter Ciliaten zur Verfügung zu stellen, gelingt auf erstaunlich einfache Art und Weise, indem man eine auxotrophe Mutante des Ciliaten herstellt, diese Mutante anschliessend mit rekombinanter DNA, die mindestens ein Gen zur Komplementierung der entsprechenden Auxotrophie enthält, transformiert und schließlich die resultierenden rekombinanten Ciliaten auf einem Minimalmedium, das nur entsprechend komplementierten Ciliaten das Wachstum ermöglicht, selektioniert, wobei Ciliaten der Genera *Paramecium* und *Tetrahymena* eingesetzt werden. Insbesondere ist für dieses Verfahren, um den Selektionsdruck dauerhaft aufrecht zu erhalten, schlussendlich weder eine Selektion auf eine Resistenz gegen ein Antibiotikum notwendig, noch auch nur die Gegenwart unerwünschter ev. heterologer Gene im rekombinanten Organismus. Eine Zugabe von Antibiotika zum Kultivierungsmedium kann somit unterbleiben. Das Verfahren läßt sich auch problemlos mehrmals auf denselben Organismen-Stamm anwenden, diesen mit verschiedensten gewünschten rekombinanten Genen transformierend, ohne jedwede (Über-) Expression weiterer ev. unerwünschter Gene.

Unter einem anderen Aspekt der vorliegenden Erfindung kann auf ebenso einfache Art und Weise ein Verfahren zur Herstellung rekombinanter Proteine zur Verfügung gestellt werden, indem man in vorgehend beschriebener Weise rekombinante Ciliaten herstellt, wobei die rekombinante DNA zur Transformation der Ciliaten zusätzlich mindestens ein funktionelles rekombinantes Gen für ein zu exprimierendes Protein umfasst. Die rekombinanten Ciliaten werden im Folgenden kultiviert, sodass die Proteine exprimiert werden und anschliessend isoliert werden können.

Bei einer bevorzugten Ausführungsform der vorliegenden Erfindung erfolgt die Herstellung der auxotrophen Mutante durch Knock-out eines essentiellen Gens. Der Knock-out kann durch vollständige Deletion des entsprechenden Gens oder aber durch Mutation desselben erreicht werden. Unter Mutationen versteht man beispielsweise Insertionen, Deletionen, Inversionen oder auch nur den Austausch einzelner Basenpaare. Gen-Deletionen bzw. Mutationen können mittels dem Fachmann bekannten Verfahren in den Zielorgansimus eingeführt werden. Unter anderem bieten sich hier *in-vitro* Mutagenese, beispielsweise durch ungenaue PCR (error-prone PCR) oder etwa nach der Kunkel-Methode (Sambrook et al. Molecular Cloning, A Laboratory Manual, Coldspring Harbor, NY) an, oder Exon-shuffling oder Gene site saturation mutagenization (GSSM) (siehe z.B.: www.Diversa.com und www.Maxygen.com)

Für die Zwecke der vorliegenden Erfindung versteht man unter essentiellen Genen für die Herstellung einer auxotrophen Knock-out Mutante essentielle Stoffwechselgene, wie z.B. der Fettsäure-, Sterol-, Aminosäurebiosynthese etc., deren Ausfall durch Zugabe der entsprechenden Moleküle (Fettsäuren, Sterole, Aminosäuren etc.) ins Kultivierungsmedium ausgeglichen werden können (dann auch Marker genannt). Durch den gezielten Knock-out solcher Gene werden die Zellen auxotroph für Produkte dieses Stoffwechselweges. In der vorliegenden Erfindung wird dies beispielhaft für den Sterol- und die Fettsäurebiosynthese beschrieben. Durch die Zugabe des entsprechenden Stoffwechselproduktes, d.h. Cholesterol, bzw. Fettsäuren können die Zellen diesen Knock-out überleben. Ohne die Zusätze sterben die Zellen rasch ab.

Gene, die beispielsweise für eine Triterpenoid-Cyclase (synonym für Tetrahymanol-Cyclase), eine delta-6-Desaturase oder eine delta-9-Desaturase codieren, sind daher erfindungsgemäß geeignete Ziele für einen Knock-out, um eine auxotrophe Mutante des respektiven Organismus herzustellen.

Ein erfindungsgemäß bevorzugtes Gen für eine Triterpenoid-Cyclase wurde in der Deutschen Patentanmeldung DE 199 57 889 A1 beschrieben. Ein erfindungsgemäß bevorzugtes Gen für eine delta-6-Desaturase wurde in der Deutschen Patentanmeldung DE 100 44 468 A1 beschrieben. Ein erfindungsgemäß bevorzugtcs Gen für eine delta-9-Desaturase wurde von Nakashima S. et al. m Biochem. J. 317, 29-34 (1996) beschrieben, die respektive Sequenz findet sich in der GenBank unter der Accession No.: EMBL D83478. Zum Thema GenBank siehe Benson, D.A. et al., Nuc. Acid Res., 28 (1), 15-18 (2000).

Erfindungsgemäß erlangen die Zellen durch das Wiedereinbringen des ausgeschalteten Gens, erneut ihre Autotrophie für dieses Stoffwechselprodukt. In diesem Falle spricht man davon, dass die Auxotrophie komplementiert wird. Die Selektion auf erfolgreich transformierte Zellen erfolgt in Minimalmedium, d. h. im Speziellen unter Weglassung des Stoffwechselproduktes, für das die nicht erfolgreich transformierten Organismen auxotroph sind. Erfindungsgemäß wird unter einem Minimalmedium ein Medium verstanden, das alle notwendigen Bausteine, die ein Überleben der Zellen ermöglichen, enthält (Kohlenstoffquelle(n), wie beispielsweise Zucker, Stickstoffquelle(n), ev. Aminosäuren, Vitamine, Spurenelemente etc.), das aber eben jenes Stoffwechselprodukt nicht enthält, für das der Ausgangsorganismus auxotroph ist.

Wird nun dieses die Auxotrophie komplementierende Gen an ein zu exprimierendes für ein heterologes Protein codierendes Gen gekoppelt, können die Transformanten ohne Zugabe eines Selektionsmarkers identifiziert werden und darüber hinaus ist die erfolgreiche stabile Expression nicht abhängig von der Zugabe eines Selektionsmarkers, wie es z. B. typischerweise bei rekombinanten *E. coli* der Fall ist. Als weiterer positiver Effekt wird, außer dem zu exprimierenden Zielgen, keinerlei Fremd-DNA in die Zelle eingebracht. Zusätzlich wird bei Organismen, bei denen homologe Rekombination stattfindet (z. B. *Tetrahymena*), die DNA nicht zufällig im Genom integriert sondern an einer bestimmten Position, nämlich der natürlichen Position des Markergens.

Es ist dem Fachmann jedoch klar, dass eine Komplementierung der Auxotrophie auch mittels entsprechender heterologer oder *in-vitro* modifizierter Gene erfolgen kann.

Erfindungsgemäß sind für zur Selektion nach dem oben beschriebenen Verfahren geeignete transformierte Ciliaten, der Genera *Paramecium* oder *Tetrahymena*, besonders bevorzugt der Spezies *Tetrahymena thermophila*.

Rekombinante DNA zur Transformation der auxotrophen Protistenmutante kann z. B. ein Vektor sein, d. h. jede Art von Nukleinsäre, wie z. B. ein Plasmid, Cosmid, Virus, eine sich autonom replizierende Sequenz, ein Phage, ein lineares oder zirkuläres, einzel- oder doppel-strängiges DNA oder RNA Molekül, das sich im Zielorganismus selbst replizieren kann oder ins Genom eingebaut werden kann, zumindest aber im Zielorganismus funktionelle Sequenzen enthält.

Erfindungsgemäß versteht man unter funktionellen Sequenzen solche DNA Abschnitte, die ihre jeweilige Funktion auch im rekombinbanten Organismus erfüllen können.

Unter einem funktionellen Gen versteht man für die Zwecke der vorliegenden Erfindung beispielsweise ein Gen, das im Zielorganismus exprimiert werden kann. Insbesondere umfasst ein funktionelles Gen daher neben einer codierenden Sequenz, einen im Zielorganismus funktionellen Promotor, der zur Transkription der codierenden Sequenz führt. Ein dergestalt funktioneller Promotor kann unter anderem eine oder mehrere TATA-Boxen, CCAAT-Boxen, GC-Boxen oder Enhancer-Sequenzen aufweisen. Weiters kann das funktionelle Gen einen im Zielorganismus funktionellen Terminator umfassen der zum Abbruch der Trankription führt und Signalsequenzen enthält, die zur Polyadenylierung der mRNA führen. Die codierende Sequenz des funktionellen Gens weist ferner alle für die Translation im Zielorgansimus notwendigen Eigenschaften auf (beispielsweise Start-Codon (z.B. ATG), Stop-Codon (z.B. TGA, im Speziellen bei *Tetrahymena*) A-reiche Regionen vor dem Start (Translation initiation sites), Kozak-Sequenzen, Poly-A Stelle. Das Gen kann auch die für den jeweiligen rekombinanten Organismus spezifische Codonusage aufweisen (für *Tetrahymena* siehe beispielsweise Wuitschick & Karrer, J. Eukaryot. Microbiol. (1999)).

Erfindungsgemäß handelt es sich bei einem rekombinanten Gen für ein in einem rekombinanten Ciliaten zu exprimierendes Protein bei einem Verfahren zur Herstellung rekombinanter Proteine um ein homologes oder ein heterologes Gen. Handelt es sich um ein heterologes Gen wird es bevorzugterweise aus einem Vertebraten isoliert, besonders bevorzugt aus dem Menschen. Ein bevorzugtes Beispiel hierfür ist menschliches Erythropoietin. Weitere erfindungsgemäß bevorzugte rekombinante Gene für in rekombinanten Ciliaten zu exprimierende Proteine sind solche aus Organismen, die beim Menschen oder bei Tieren Krankheiten auslösen können (wie z. B.: Malaria), um mit Hilfe der rekombinanten Proteine oder auch der die rekombinanten Proteine enthaltenden Biomasse oder Teilen davon, eine aktive Immunisierung erreichen zu können.

Beschreibung der Abbildungen:
Abb. 1: Struktur des Tetrahymanol-Gens pgTHC
Abb. 2: Tetrahymanol-Knock-out Konstrukt pgTHC::neo
Abb. 3: Expressionskonstrukt pBTHC
Abb. 4: Struktur des Tetrahymanol-Cyclase/neo Konstruktes pgTHC+neo
Abb. 5: Delta-6 Desaturase Knock-out Konstrukt pgDES6::neo
Abb. 6: Struktur des genomischen delta-6 Desaturase-Gens pgDES6
Abb. 7: Struktur des delta-6 Desaturase/neo Konstruktes pgDES6+neo

Die nachfolgenden Beispiele dienen zur Erläuterung der Erfindung, ohne die Erfindung auf diese Beispiele einzugrenzen.

### BEISPIEL 1: Organismen und Kultivierungsbedingungen

*Tetrahymena thermophila* (Stämme B1868 VII, B2086 II, B*VI, CU428, CU427, CU522, zur Verfügung gestellt von Dr. J. Gaertig, University of Georgia, Athens, GA, USA) wurden in modifiziertem SPP-Medium (2% Proteosepeptone, 0,1% Hefeextrakt, 0,2% Glucose, 0,003% Fe-EDTA (Gaertig et al. (1994) PNAS 91:4549-4553)) bzw. Magermilchmedium (2% Magermilchpulver, 0,5% Hefeextrakt, 1% Glucose, 0,003% Fe-EDTA) oder MYG-Medium (2% Magermilchpulver, 0,1% Hefeextrakt, 0,2% Glucose, 0,003% Fe-EDTA) mit Zusatz von Antibiotika-Lösung (100 U/ml Penicillin, 100 µg/ml Streptomycin und 0,25 µg/ml Amphotericin B (SPPA-Medium)) bei 30 °C in 50 ml Volumen in 250 ml Erlenmeyerkolben unter Schütteln (150 U/min) kultiviert.

Plasmide und Phagen wurden in *E. coli* XL1-Blue MRF', TOP10F' oder JM109 (Stratagene, Invitrogen, GibcoBRL Life Technologies) vermehrt und selektiert. Die Kultivierung der Bakterien erfolgte unter Standardbedingungen in LB- oder NZY-Medium, mit Antibiotika in Standardkonzentrationen (Sambrook et al. (1989) Molecular Cloning: A Laboratory Manual, Cold Spring Harbor Laboratory, Cold Spring, New York).

### BEISPIEL 2: Herstellung des Triterpenoid-Cyclase Knock-out Konstruktes

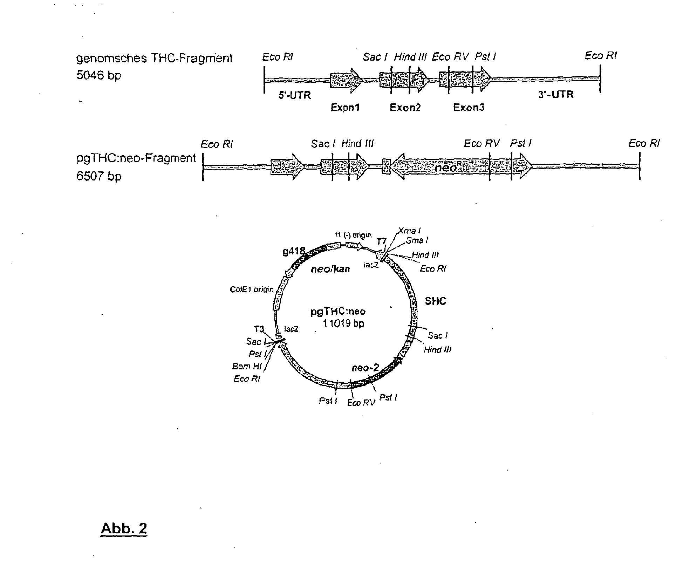

Für die Herstellung des Knock-out Konstruktes wurde in die genomische Sequenz der Triterpenoid-Cyclase (Patentanmeldung DE 199 57 889 A1) eine neo-Kassette aus dem Plasmid p4T2-1ΔH3 (Gaertig et al. (1994) Nucl. Acids Res. 22:5391-5398) inseriert. Dabei handelt es sich um das Neomycin-Resistenzgen unter der Kontrolle des *Tetrahymena* Histon H4-Promoters und der 3' flankierenden Sequenz des BTU2 Gens. Dieses Konstrukt vermittelt in *Tetrahymena* eine Resistenz gegenüber Paromomycin. Das Plasmid p4T2-1ΔH3 wurde mit Eco RV/Sma I geschnitten und das ca. 1,4 kb große Fragment, umfassend die neo-Kassette wurde in das mit Eco RV geschnittene Plasmid pgTHC in die genomische Sequenz der Tetrahymena Triterpenoid-Cyclase ligiert. Dadurch wurde das Plasmid pgTHC::neo erzeugt (s. Abb. 2). Bei einer erfolgreichen Transformation wurde durch homologe Rekombination das Gen für die Triterpenoid-Cyclase durch dieses Konstrukt ersetzt, wodurch eine Resistenz der Zellen gegenüber Paromomycin vermittelt wurde.

### BEISPIEL 3: Herstellung des Expressionskonstruktes pBTHC

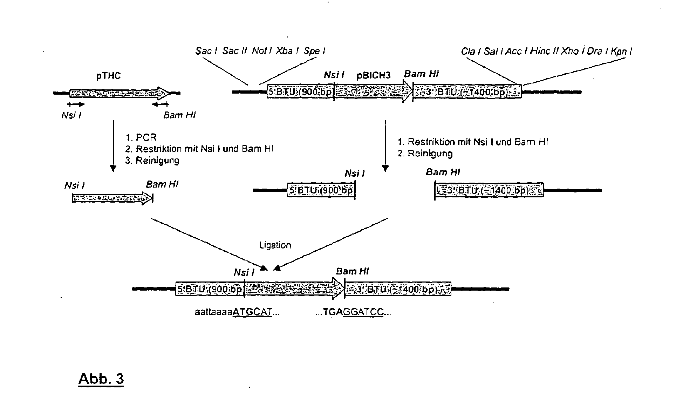

Der Vektor pBICH3 (Gaertig et al. 1999 Nature Biotech. 17: 462-465, WO 00/46381) enthält die kodierende Sequenz des Ichthyophthirius I-Antigen (G1) Präprotein flankiert von den nicht kodierenden, regulativen Sequenzen des *Tetrahymena thermophila* BTU1-Gens. Ein modifiziertes Plasmid (pBICH3-Nsi) mit einer Nsi I Schnittstelle am Start (zur Verfügung gestellt von J. Gaertig, University of Georgia, Athens, GA, USA) wurde benutzt, um das Tetrahymanol-Cyclase Expressionskonstrukt pBTHC herzustellen. Dazu wurden mittels PCR Nsi I und Bam HI Schnittstellen am Start und Stop der kodierenden Sequenzen der Tetrahymanol-Cyclase von Tetrahymena eingefügt. Für die PCR wurden isolierte Plasmide, welche die vollständigen cDNA-Sequenzen der Tetrahymanol-Cyclase enthalten (pTHC) als Template eingesetzt. Die Primer
THC-Nsi-F: 5'- CTCTTTCATACATGCATAAGATACTCATAGGC-3' (SEQ ID No 1) und
THC-Bam-R: 5'- GGCTTGGATCCTCAAATATTTTATTTTTATACAGG-3' (SEQ ID No 2)
erzeugten PCR-Produkte, welche die vollständige kodierende Sequenz der Tetrahymanol-Cyclase, flankiert von Nsi I und Bam HI Schnittstellen, enthielten. Die PCR-Produkte und das Plasmid pBICH3-Nsi wurden mit den Restriktionsenzymen Nsi I und Bam HI geschnitten, über ein Agarosegel gereinigt und ligiert (s. Abb. 3). Das so entstandene Expressionskonstrukt pBTHC enthielt die vollständige die Triterpenoid-Cyclase kodierende Sequenz inseriert in einem korrekten Leseraster in die regulatorischen Sequenzen des BTU1 Gens. Für die Transformation von *Tetrahymena* wurden die Konstrukte durch einen Verdau mit den Restriktionsenzymen Xba I und Sal I linearisiert.

Bei einer erfolgreichen Transformation wurde durch homologe Rekombination das BTU1-Gen durch diese Konstrukte ersetzt, wodurch eine Resistenz der Zellen gegenüber Paclitaxel vermittelt wurde.

### BEISPIEL 4: Macronucleus-Transformation von Tetrahymena mit dem Tetrahymanol-Cyclase Expressionskonstrukt pBTHC

Für eine Transformation wurden 5 x 10⁶ *Tetrahymena thermophila* Zellen (CU522) eingesetzt. Die Kultivierung der Zellen erfolgte in 50 ml SPPA-Medium bei 30 °C in einem 250 ml Erlenmeyerkolben auf einem Schüttler bei 150 RPM bis zu einer Zelldichte von ca. 3-5 x 10⁵ Zellen/ml. Die Zellen wurden durch Zentrifugation (1200 g) für 5 min pelletiert und das Zellpellet wurde in 50 ml 10 mM Tris-HCl (pH 7,5) resuspendiert und wie vorher zentrifugiert. Dieser Waschschritt wurde wiederholt und die Zellen in 10 mM Tris-HCl (pH 7,5, plus Antibiotika) bei einer Zelldichte von 3 x 10⁵ Zellen/ml resuspendiert, in einen 250ml Erlenmeyerkolben überführt und für 16-20 h ohne Schütteln bei 30 °C inkubiert (Hungerphase). Nach der Hungerphase wurde erneut die Zellzahl bestimmt, wie oben zentrifugiert und die Zellen mit 10 mM Tris-HCl (pH 7,5) auf eine Konzentration von 5 x 10⁶ Zellen/ml eingestellt. Ein ml der Zell-Suspension wurde für die Transformation benutzt. Die Transformation erfolgte mittels Mikropartikelbeschuss (siehe unten). Zur Regeneration wurden die Zellen in SSPA-Medium aufgenommen und bei 30 °C ohne Schütteln im Erlenmeyerkolben inkubiert. Nach 3 h wurde Paclitaxel® in einer Endkonzentration von 20 µM zugegeben und die Zellen in Aliquots von 100 µl auf 96er Mikrotiterplatten überführt. Die Zellen wurden in einer feuchten, abgedunkelten Box bei 30 °C inkubiert. Nach 2-3 Tagen konnten Paclitaxel-resistente Klone identifiziert werden. Positive Klone wurden in frisches Medium mit 25 µM Paclitaxel überimpft. Durch Kultivierung der Zellen in steigender Paclitaxel-Konzentration (bis 80 µM) wurde ein komplettes "phenotypic assortment" (Gaertig & Kapler (1999)) erreicht.

Zur Analyse der Klone wurden ca. 4 ml Kulturen in SPPA mit Paclitaxel angezogen, DNA isoliert (Jacek Gaertig et al. (1994) PNAS 91:4549-4553) und durch PCR die in den BTU1-Locus integrierte DNA amplifiziert. Als Primer dienten die BTU1 spezifischen Primer

BTUI-5'F (AAAAATAAAAAAGTTTGAAAAAAAACCTTC (SEQ ID No 3), ca 50 bp vor dem Startcodon und BTU1-3'R (GTTTAGCTGACCGATTCAGTTC (SEQ ID No 4), 3 bp hinter dem Stopcodon. Die PCR-Produkte wurden ungeschnitten und mit Hind III, Sac I oder Pst I geschnitten auf einem 1%igen Agarosegel analysiert. Vollständiges "phenotypic assortment" wurde über RT-PCR mit den BTU1 spezifischen Primern (Gaertig & Kapler (1999)) überprüft.

### BEISPIEL 5: Herstellung des delta-6-Desaturase Knock-out Konstruktes pgDES6::neo

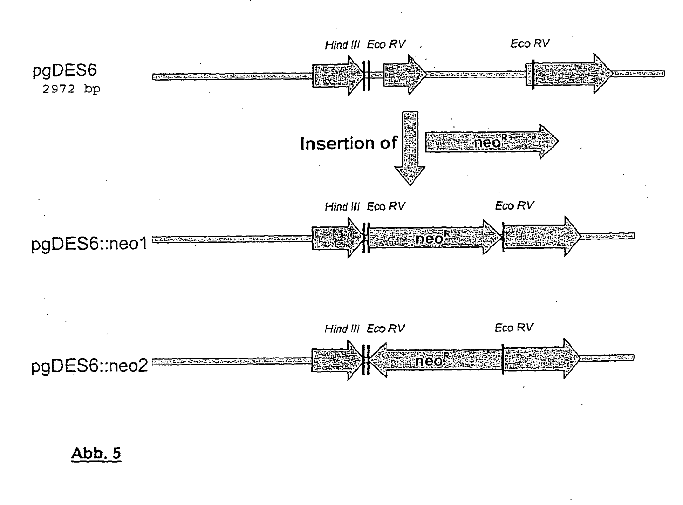

Für die Herstellung des Knock-out Konstruktes wurde in die genomische Sequenz der delta-6-Desaturase eine neo-Kassette aus dem Plasmid p4T2-1ΔH3 (Patentanmeldung DE 100 44 468 A1) inseriert. Dabei handelt es sich um das Neomycin-Resistenzgen unter der Kontrolle des *Tetrahymena* Histon H4-Promoters und der 3' flankierenden Sequenz des BTU2 Gens. Dieses Konstrukt vermittelt in *Tetrahymena* eine Resistenz gegenüber Paromomycin. Das Plasmid P4T2-1ΔH3 wurde mit Eco RV/Sma I geschnitten und das ca. 1,4 kb große Fragment der neo-Kassette wurde in das mit Eco RV geschnittene Plasmid pgDES6 (Patentanmeldung DE 100 44 468 A1) in die genomische Sequenz der *Tetrahymena* delta-6-Desaturase ligiert (s. Abb. 5). Dadurch wurde das Plasmid pgDES6::neo erzeugt. Bei einer erfolgreichen Transformation wurde durch homologe Rekombination das Gen für die delta-6-Desaturase durch dieses Konstrukt ersetzt, wodurch eine Resistenz der Zellen gegenüber Paromomycin vermittelt wurde.

### BEISPIEL 6: Micronucleus- und Macronucleus-Transformation von Tetrahymena mit den Knock-out Konstrukten pgTHC::neo und pgDES6::neo

*Tetrahymena* Stämme unterschiedlichen Paarungstyps (CU428 VII und B2086 II) wurden getrennt in SPPA-Medium bei 30 °C unter Schütteln (150 Upm) in Erlenmeyerkolben kultiviert. Bei einer Zelldichte von 3-5 x 10⁵ Zellen/ml wurden die Zellen 5 min bei Raumtemperatur zentrifugiert (1200 g). Die Zellen wurden dreimal mit 50 ml 10 mM Tris-HCl (pH 7,5) gewaschen und schließlich in 50 ml 10 mM Tris-HCl (pH 7,5) resuspendiert und mit Antibiotika-Lösung versetzt und daraufhin ohne Schütteln in Erlenmeyerkolben bei 30 °C inkubiert. Nach ca. 4 h wurde die Zellzahl beider Kulturen erneut bestimmt und mit 10 mM Tris-HCl (pH 7,5) auf 3 x 10⁵ Zellen/ml eingestellt. Daraufhin wurden die Kulturen für weitere 16-20 h bei 30 °C inkubiert. Nach dieser Hungerphase wurde von beiden Kulturen die gleiche (absolute) Zellzahl in einem 2-L Erlenmeyerkolben gemischt. Die Zellen wurden bei 30 °C inkubiert (Beginn der Konjugation) und nach 2 h wurde die Effizienz der Konjugation bestimmt. Für eine erfolgreiche Transformation sollten zu diesem Zeitpunkt ca. 30% der Zellen als Paare vorliegen.

Für die Micronucleus-Transformation wurden 3 h, 3,5 h, 4 h und 4,5 h nach Beginn der Konjugation jeweils 1 x 10⁷ konjugierende Zellen (5 x 10⁶ Paare) 5 min bei 1200 g zentrifugiert und das Zellpellet in 1 ml 10 mM Tris-HCl (pH 7,5) resuspendiert.

Für die Transformation der neuen Macronucleus-Anlagen wurden 11 h nach Beginn der Konjugation Zellen wie oben zentrifugiert und in Tris-HCl resuspendiert.

Die Transformation erfolgte mittels Mikropartikelbeschuss (s. u.).

Für die Kultivierung der Tetrahymanol-Cyclase Knock-out-Mutanten wurden 10 µg/ml Cholesterol zum Medium zugegeben.

Für die Kultivierung der delta-6-Desaturase Knock-out-Mutanten wurden 200 µg/ml Borage-Öl (20-25% GLA; SIGMA) zum Medium zugegeben.

Durch Selektion auf Paromomycinresistenz konnten transformierte Zellen identifiziert werden. Bei der Transformation des Mikronucleus wurde 11 h nach Beginn der Konjugation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Echte Mikronucleus-Transformanten konnten anhand der Resistenz gegenüber 6-Methylpurin von Macronucleus-Transformanten unterschieden werden. Bei der Transformation des Macronucleus wurde ca. 4 h nach der Transformation Paromomycin (100 µg/ml Endkonzentration) zugegeben und die Zellen in Aliquots von 100 µl auf 96er Mikrotiterplatten verteilt. Die Zellen wurden in einer feuchten Box bei 30 °C inkubiert. Nach 2-3 Tagen konnten resistente Klone identifiziert werden. Positive Klone wurden in frisches Medium mit 120 µg/ml Paromomycin überimpft. Durch Kultivierung der Zellen in dieser hohen Paromomycin-Konzentration wurde nach einigen Generationen ein komplettes "phenotypic assortment" (Gaertig & Kapler (1999)) erreicht.

Durch Kreuzung der Micronucleus-Transformanten mit einem B*VI-Stamm konnten homozygote Knock-out-Mutanten erzeugt werden (Bruns & Cassidy-Hanley, Methods in Cell Biology, Volume 62 (1999) 229-240).

### BEISPIEL 7: Biolistische Transformation (Mikropartikelbeschuss)

Die Transformation von *Tetrahymena thermophila* erfolgte mittels biolistischer Transformation, wie sie bei Bruns & Cassidy-Hanley (Methods in Cell Biology, Volume 62 (1999) 501-512); Gaertig et al. (1999) Nature Biotech. 17: 462-465) oder Cassidy-Hanley et al. ((1997) Genetics 146:135-147) beschrieben ist. Die Handhabung des Biolistic® PDS-1000/He Particle Delivery System (BIO-RAD) ist detailiert im zugehörigen Handbuch beschrieben.

Für die Transformation werden 6 mg Goldpartikel (0,6 µm; BIO-RAD) mit 10 µg linearisierter Plasmid DNA beladen (Sanford et al. (1991) Biotechniques 3:3-16; Bruns & Cassidy-Hanley (1999) Methods in Cell Biology, Volume 62: 501-512).

Vorbereitung der Goldpartikel: 60 mg der 0,6 µm Goldpartikel (Biorad) wurden in 1 ml Ethanol resuspendiert. Dazu wurden die Partikel 3 mal für je 1-2 min auf einem Vortex kräftig gemixt. Anschließend wurden die Partikel für 1 min zentrifugiert (10000 g) und der Überstand vorsichtig mit einer Pipette abgenommen. Die Goldpartikel wurden in 1 ml sterilem Wasser resuspendiert und wie oben zentrifugiert. Dieser Waschschrill wurde einmal wiederholt, die Partikel in 1 ml 50%igem Glycerol resuspendiert und in Aliquots zu 100 µl bei -20 °C gelagert.

Vorbereiten der Transformation: Die Macrocarrierhalter, Macrocarrier und Stopscreens wurden für mehrere Stunden in 100% Ethanol, die Rupture Disks in Isopropanol gelagert. Ein Macrocarrier wurde anschließend in den MacrocarrierHalter eingesetzt und an der Luft getrocknet.

Beladung der Goldpartikel mit DNA: Alle Arbeiten erfolgten bei 4 °C. Goldpartikel, vorbereiteter Vektor, 2,5 M CaCl₂, 1 M Spermidine, 70% und 100% Ethanol wurden auf Eis gekühlt. 10 µl der linearisierten Vektor-DNA (1 µg/ml) wurden zu 100 µl vorbereiteten Goldpartikeln gegeben und für 10 sec vorsichtig gevortext. Anschließend wurden erst 100 µl 2,5 M CaCl₂ zugegeben, 10 sec gevortext und dann 40 µl 1 M Spermidine zugegeben und 10 min vorsichtig gevortext. Nach Zugabe von 200 µl 70%igem Ethanol wurden die Partikel für 1 min gevortext und dann 1 min bei 10000 g zentrifugiert. Das Pellet wurde in 20 µl 100%igem Ethanol resuspendiert, zentrifugiert und dann in 35 µl 100%igem Ethanol resuspendiert.

Die so vorbereiteten Partikel wurden vorsichtig mit einer Pipette auf das Zentrum eines Macrocarriers gegeben. Der Macrocarrierer wurde anschließend bis zur Transformation in einer Box mit hygroskopischen Silicagel gelagert.

Transformation: Ein ml der vorbereiteten Zellen (siehe oben) wurde in die Mitte eines mit 10 mM Tris-HCl (pH 7,5) angefeuchteten Rundfilters in einer Petrischale gegeben und in die unterste Einschubleiste der Transformationskammer des Biolistic® PDS-1000/He Particle Delivery Systems eingesetzt. Die Transformation erfolgte mit den vorbereiteten Goldpartikeln bei einem Druck von 900 psi (zwei 450 psi Rupture Disks) und einem Vacuum von 27 inches Hg in der Transformationskammer. Anschließend wurden die Zellen sofort in einen Erlenmeyerkolben mit 50 ml SPPA-Medium überführt und bei 30 °C ohne Schütteln inkubiert.

### BEISPIEL 8: Wiederherstellungs-Transformation von Tetrahymanol Knock-out Mutanten mit dem genomischen Tetrahymanol-Cyclase Plasmid pgTHC

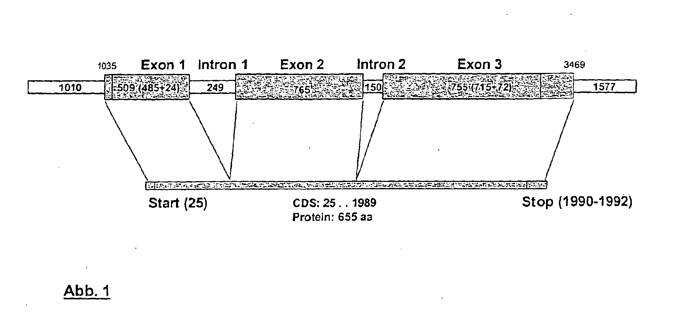

Die Transformation erfolgte analog zu Bsp. 4. Für die Transformation wurden Tetrahymanol-Knock-out Mutanten (s. Bsp. 2) von *Tetrahymena thermophila* eingesetzt. Die Kultivierung der Zellen erfolgte in SPPA-Medium mit Zusatz von 10 mg/l Cholesterol. Nach der Transformation (s.o.) mit dem genomischen Fragment der Tetrahymanolcyclase (s. Abb. 1) wurden die Zellen in SPPA-Medium ohne Cholesterol aufgenommen und bei 30 °C ohne Schütteln im Erlenmeyerkolben inkubiert. Nach 3 h wurden die Zellen in Aliquots von 100 µl auf 96er Mikrotiterplatten überführt. Die Zellen wurden in einer feuchten, abgedunkelten Box bei 30 °C inkubiert. Nach etwa 2-3 Tagen konnten die ersten Cholesterolautotrophen Klone identifiziert werden. Nach ca. 5-7 Tagen wurden die positiven Klone in frisches Medium überimpft. Durch tägliches Umimpfen der Zellen über einen Zeitraum von ca. 2 Wochen und mehrfaches isolieren von Einzellzellen wurde ein komplettes "phenotypic assortment" (Gaertig & Kapler (1999)) erreicht. Diese Klone wurden zur Kontrolle in SPPA-Medium unter Zusatz von Paromomycin kultiviert. Nach ca. 3-5 Tagen starben alle Kulturen ab. D. h. die Klone hatten ihre Resistenz gegenüber Paromomycin verloren, was im Verlust des Neo-Gens durch die homologe Rekombination begründet ist.

### BEISPIEL 9: Wiederherstellungs-Transformation von delta-6 Desaturase Knock-out Mutanten mit dem delta-6 Plasmid pgDES6

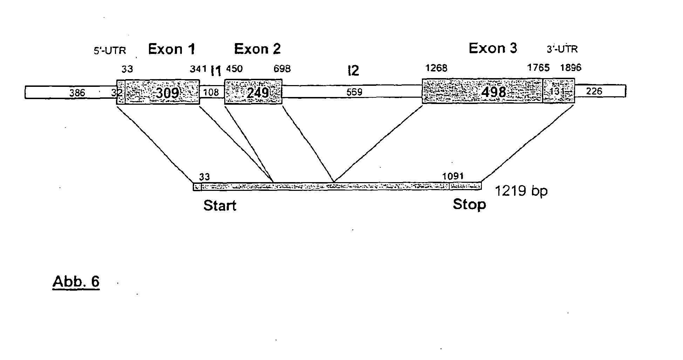

Die Transformation erfolgte analog zu Bsp. 4. Für die Transformation wurden delta-6 Desaturase-Knock-out Mutanten (s. Bsp. 5) von *Tetrahymena thermophila* eingesetzt. Die Kultivierung der Zellen erfolgte in SPPA-Medium mit Zusatz von 200µg/ml Borage-Öl (20-25% GLA; SIGMA). Nach der Transformation (s.o.) mit dem genomischen delta-6 Desaturase DNA-Fragment (s. Abb. 6) wurden die Zellen in SPPA-Medium ohne Borage-Öl aufgenommen und bei 30 °C ohne Schütteln im Erlenmeyerkolben inkubiert. Nach 3 h wurden die Zellen in Aliquots von 100 µl auf 96er Mikrotiterplatten überführt. Die Zellen wurden in einer feuchten, abgedunkelten Box bei 30 °C inkubiert. Nach etwa 2-3 Tagen konnten die ersten GLA-autotrophen Klone identifiziert werden. Nach ca. 5-7 Tagen wurden die positiven Klone in frisches Medium überimpft. Durch tägliches Umimpfen der Zellen über einen Zeitraum von ca. 2 Wochen und mehrfaches isolieren von Einzellzellen wurde ein komplettes "phenotypic assortment" (Gaertig & Kapler (1999)) erreicht. Diese Klone wurden zur Kontrolle in SPPA-Medium unter Zusatz von Paromomycin kultiviert. Nach ca. 3-5 Tagen starben alle Kulturen ab. D. h. die Klone hatten ihre Resistenz gegenüber Paromomycin verloren, was im Verlust des Neo-Gens durch die homologe Rekombination begründet ist.

### BEISPIEL 10: Wiederherstellungs-Transformation mit dem Plasmid pgTHC gekoppelt mit dem Neomycin-Gen

Die Transformation erfolgte analog zu Bsp. 8. Es wurde ein Vektor eingesetzt, der wie folgt aufgebaut war:

Das genomische Fragment der Tetraymanol-Cyclase (pgTHC) wurde mit dem Restriktionsenzym Bgl II geschnitten. Dieses Enzym schneidet das genomische Fragment außerhalb der kodierenden Exons an Position 4537 im 3'-untranslatierten Bereich. Nach Inkubation mit T4 DNA-Polymerase zum glätten der Enden wurde die Neomycin-Kassette in das Plasmid ligiert. Dazu wurde das Plasmid p4T2-1ΔH3 mit Eco RV/Sma I geschnitten und das ca. 1,4 kb große Fragment, umfassend die neo-Kassette, wurde in das bereits geschnittene Plasmid pgTHC in die 3'-untranslatierte Sequenz der Tetrahymena Triterpenoid-Cyclase ligiert.

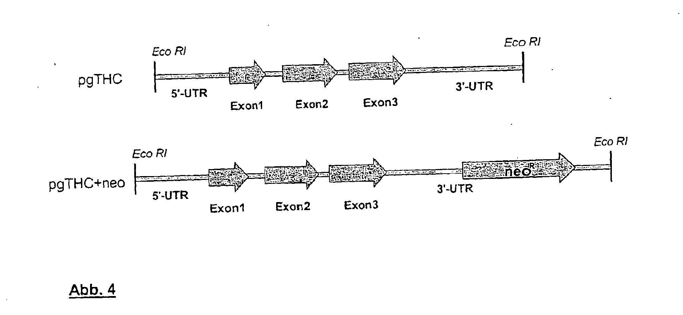

Dieses Konstrukt (pgTHC+neo, s. Abb. 4) wurde für die Transformation der Tetrahymanol-Mutanten benutzt. Nach der Transformation (s.o.) wurden die Zellen in SPPA-Medium ohne Cholesterol aufgenommen und bei 30 °C ohne Schütteln im Erlenmeyerkolben inkubiert. Nach 3 h wurde Paromomycin zugegeben und die Zellen in Aliquots von 100 µl auf 96er Mikrotiterplatten überführt. Die Zellen wurden in einer feuchten, abgedunkelten Box bei 30 °C inkubiert. Nach etwa 2-3 Tagen konnten die ersten cholesterolautotrophen Klone identifiziert werden, die gleichzeitig Paromomycin resistent waren. Die positiven Klone wurden in frisches Medium ohne Zusatz von Cholesterol oder Paromomycin überimpft. Durch tägliches Umimpfen der Zellen über einen Zeitraum von ca. 2 Wochen und mehrfaches isolieren von Einzellzellen wurde ein komplettes "phenotypic assortment" (Gaertig & Kapler (1999)) erreicht. Diese Zellen zeigten gutes Wachstum auch nach Zugabe von Paromomycin.

### BEISPIEL 11: Wiederherstellungs-Transformation mit dem Plasmid pgDES6 gekoppelt mit dem Neomycin-Gen

Die Transformation erfolgte analog zu Bsp. 9. Es wurde ein Vektor eingesetzt, der wie folgt aufgebaut war:

Das genomische Fragment der delta-6-Desaturase (pgDES6) wurde mit dem Restriktionsenzym Sna BI geschnitten. Dieses Enzym schneidet das genomische Fragment außerhalb der kodierenden Exons an Position 747 im 5'-untranslatierten Bereich. Das Plasmid p4T2-1ΔH3 wurde mit Eco RV/Sma I geschnitten und das ca. 1,4 kb große Fragment, umfassend die neo-Kassette, wurde in das bereits geschnittene Plasmid pgDES6 in die 5'-untranslatierte Sequenz der Tetrahymena delta-6 Desaturase ligiert.

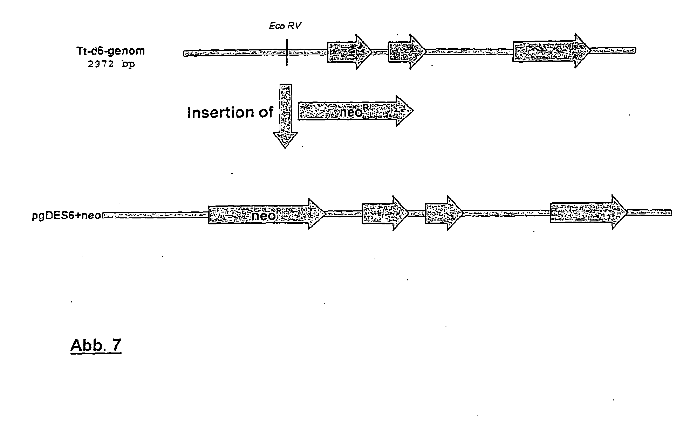

Dieses Konstrukt (pgDES6+neo, s. Abb. 7) wurde für die Transformation der delta-6 Desaturase-Mutanten benutzt. Nach der Transformation (s.o.) wurden die Zellen in SPPA-Medium ohne Borage-Öl aufgenommen und bei 30 °C ohne Schütteln im Erlenmeyerkolben inkubiert. Nach 3 h wurde Paromomycin zugegeben und die Zellen in Aliquots von 100 µl auf 96er Mikrotiterplatten überführt. Die Zellen wurden in einer feuchten, abgedunkelten Box bei 30 °C inkubiert. Nach etwa 2-3 Tagen konnten die ersten Cholesterolautotrophen Klone identifiziert werden, die gleichzeitig Paromomycin resistent waren. Die positiven Klone wurden in frisches Medium ohne Zusatz von Cholesterol oder Paromomycin überimpft. Durch tägliches Umimpfen der Zellen über einen Zeitraum von ca. 2 Wochen und mehrfaches isolieren von Einzellzellen wurde ein komplettes "phenotypic assortment" (Gaertig & Kapler (1999)) erreicht. Diese Zellen zeigten gutes Wachstum auch nach Zugabe von Paromomycin.

## Patentansprüche

1. Verfahren zur Herstellung rekombinanter Ciliaten, umfassend die Schritte:
a) Herstellung einer auxotrophen Mutante des Ciliaten,
b) Transformation der Mutante mit rekombinanter DNA, mindestens enthaltend ein Gen zur Komplementierung der entsprechenden Auxotrophie,
c) Selektion der rekombinanten Ciliaten auf einem Minimalmedium, das nur entsprechend komplementierten Ciliaten das Wachstum ermöglicht,
wobei Ciliaten der Genera *Paramecium* und *Tetrahymena* eingesetzt werden.

2. Verfahren nach Anspruch 1, wobei die Herstellung der auxotrophen Mutante durch Knock-out eines essentiellen Gens erfolgt.

3. Verfahren nach Anspruch 2, wobei das ausgeknockte Gen für eine Triterpenoid-Cyclase, eine delta-6-Desaturase oder eine delta-9-Desaturase codiert.

4. Verfahren nach einem der Ansprüche 1 bis 3, wobei die Ciliaten den Genera Paramecium oder Tetrahymena, bevorzugt der Spezies Tetrahymena thermophila, angehören.

5. Verfahren nach einem der Anspruche 1 bis 4, wobei die rekombinante DNA zur Transformation der auxotrophen Ciliatenmutante ein Gen für eine Triterpenoid-Cyclase, eine delta-6-Desaturase oder eine delta-9- Desaturase enthält.

6. Verfahren zur Herstellung rekombinanter Proteine umfassend die Stufen:
a) Herstellung rekombinanter Ciliaten gemäß des Verfahrens nach einem der Ansprüche 1-5, wobei die rekombinante DNA zur Transformation der Ciliaten zusätzlich mindestens ein funktionelles rekombinantes Gen für ein zu exprimierendes Protein enthält,
b) Kultivierung der rekombinaten Ciliaten und Expression der Proteine,
c) Isolation der Proteine.

7. Proteinherstellungsverfahren nach Anspruch 6, wobei das rekombinante Gen für das zu exprimierende Protein aus einem Vertebraten isoliert wurde, bevorzugt aus dem Menschen.

## Claims

1. Method for production of recombinant ciliates, comprising the steps:
a) production of an auxotrophic mutant of the ciliate,
b) transformation of the mutant with recombinant DNA, containing at least one gene for complementation of the corresponding auxotrophy,
c) selection of the recombinant ciliates on a minimal medium that permits growth only of the corresponding complemented ciliates,
wherein ciliates of the genera *Paramecium* and *Tetrahymena* are used.

2. Method according to claim 1, in which production of the auxotrophic mutants occurs by knockout of an essential gene.

3. Method according to claim 2, in which the knocked out gene codes for a triterpenoid-cyclase, a delta-6-desaturase or a delta-9-desahirase,

4. Method according to anyone of the claims 1-3, in which the ciliates belong to the genera *Paramecium* or *Tetrahymena,* preferably to the species Tetrahymena thermophila.

5. Method according to anyone of the claims 1-4, in which the recombinant DNA for transformation of the auxotrophic ciliate mutant contains a gene for a triterpenoid-cyclase, a delta-6-desaturase or a delta-9-desaturase.

6. Method for production of recombinant proteins, comprising the stages:
a) production of recombinant ciliates according to the method of anyone of the claims 1-5, wherein the recombinant DNA for transformation of the ciliates additionally contains at least one functional recombinant gene for a protein, which is to be expressed,
b) culturing of the recombinant ciliates and expression of the proteins,
c) isolation of the proteins.

7. Protein production method according to claim 6, wherein the recombinant gene for the protein to be expressed was isolated from a vertebrate, preferably from a human.

## Revendications

1. Procédé de production de ciliés recombinants, comprenant les étapes consistant à :
a) produire un mutant auxotrophe du cilié,
b) transformer le mutant à l'aide d'ADN recombinant, contenant au moins un gène de complémentation de l'auxotrophie correspondante,
c) sélectionner les ciliés recombinants sur un milieu minimal ne permettant que la croissance des ciliés complémentés de manière correspondante,
des ciliés des genres *Paramecium* et *Tetrahymena* étant utilisés.

2. Procédé selon la revendication 1, dans lequel la production des mutants auxotrophes s'effectue par l'invalidation d'un gène essentiel.

3. Procédé selon la revendication 2, dans lequel le gène invalidé code une triterpénoïde cyclase, une delta-6-désaturase ou une delta-9-désaturase.

4. Procédé selon l'une des revendications 1 à 3, dans lequel les ciliés appartienent aux genres Paramecium ou Tetrahymena, de préférence à l'espèce Tetrahymena thermophila.

5. Procédé selon l'une des revendications 1 à 4, dans lequel l'ADN recombinant pour la transformation du mutant de cilié auxotrophe contient un gène pour une triterpénoïde cyclase, une delta-6-désaturase ou une delta-9-désaturase.

6. Procédé de production de protéines recombinantes comprenant les étapes consistant à :
a) produire des ciliés recombinants conformément au procédé selon l'une des revendications 1 à 5, dans lequel l'ADN recombinant destiné à la transformation des ciliés contient en outre au moins un gène recombinant fonctionnel pour une protéine à exprimer,
b) cultiver les ciliés recombinants et l'expression des protéines,
c) isoler les protéines.

7. Procédé de production de protéines selon la revendication 6, dans lequel le gène recombinant pour la protéine à exprimer a été isolé à partir d'un vertébré, de préférence de l'homme.
